# EUROPEAN PATENT SPECIFICATION

(11) **EP 2 994 125 B1**
(45) Date of publication and mention of the grant of the patent: **01.08.2018**
(21) Application number: 14721905.9
(22) Date of filing: 08.05.2014
(51) Int. Cl.: A61K 31/337, A61K 31/496, A61K 31/519, A61K 45/06, A61P 35/00

(54) **NINTEDANIB ZUR ANWENDUNG IN DER BEHANDLUNG VON NSCLC**
NINTEDANIB FOR USE IN THE TREATMENT OF NSCLC
NINTEDANIB POUR UTILISATION DANS LE TRAITEMENT DU NSCLC

(30) Priority: 10.05.2013 EP 13167358
(43) Date of publication of application: 16.03.2016
(73) Proprietor: Boehringer Ingelheim International GmbH, 55216 Ingelheim am Rhein (DE)
(72) Inventor: GASCHLER-MARKEFSKI, Birgit, 55216 Ingelheim Am Rhein (DE); KAISER, Rolf, 55216Ingelheim Am Rhein (DE)
(74) Representative: Simon, Elke Anna Maria
(86) International application number: PCT/EP2014/059456
(87) International publication number: WO 2014/180955

(56) References cited:
- WO-A1-2009/147218
- MARTIN RECK ET AL: "Docetaxel plus nintedanib versus docetaxel plus placebo in patients with previously treated non-small-cell lung cancer (LUME-Lung 1): a phase 3, double-blind, randomised controlled trial", THE LANCET ONCOLOGY, vol. 15, no. 2, 1 February 2014 (2014-02-01), pages 143-145, XP055126729,
- Nasser H Hanna ET AL: "Lume-lung 2: A multicenter, randomized, double-blind, phase III study of nintedanib plus pemetrexed versus placebo plus pemetrexed in patients with advanced nonsquamous non-small cell lung cancer (NSCLC) after failure of first-line chemotherapy.", J Clin Oncol 31, 2013 (suppl; abstr 8034), 30 May 2013 (2013-05-30), XP055126749, Retrieved from the Internet: URL:http://meetinglibrary.asco.org/print/1 167801 [retrieved on 2014-07-03]
- M. RECK ET AL: "A phase II double-blind study to investigate efficacy and safety of two doses of the triple angiokinase inhibitor BIBF 1120 in patients with relapsed advanced non-small-cell lung cancer", ANNALS OF ONCOLOGY, vol. 22, no. 6, 6 January 2011 (2011-01-06), pages 1374-1381, XP055126764, ISSN: 0923-7534, DOI: 10.1093/annonc/mdq618
- P. M. ELLIS ET AL: "Phase I Open-Label Study of Continuous Treatment with BIBF 1120, a Triple Angiokinase Inhibitor, and Pemetrexed in Pretreated Non-Small Cell Lung Cancer Patients", CLINICAL CANCER RESEARCH, vol. 16, no. 10, 11 May 2010 (2010-05-11), pages 2881-2889, XP055126754, ISSN: 1078-0432, DOI: 10.1158/1078-0432.CCR-09-2944
- STOPFER P ET AL: "9152 A population pharmacokinetic analysis for BIBF 1120, an angiokinase inhibitor, in patients with advanced non-small cell lung cancer", EUROPEAN JOURNAL OF CANCER. SUPPLEMENT, PERGAMON, OXFORD, GB, vol. 7, no. 2, 1 September 2009 (2009-09-01), page 551, XP026690671, ISSN: 1359-6349, DOI: 10.1016/S1359-6349(09)71865-8 [retrieved on 2009-09-01]
- EDGARDO S SANTOS ET AL: "Targeting angiogenesis from multiple pathways simultaneously: BIBF 1120, an investigational novel triple angiokinase inhibitor", INVESTIGATIONAL NEW DRUGS ; THE JOURNAL OF NEW ANTICANCER AGENTS, KLUWER ACADEMIC PUBLISHERS, BO, vol. 30, no. 3, 25 February 2011 (2011-02-25), pages 1261-1269, XP035052857, ISSN: 1573-0646, DOI: 10.1007/S10637-011-9644-2
- KEUN-WOOK LEE ET AL: "Weekly Low-Dose Docetaxel for Salvage Chemotherapy in Pretreated Elderly or Poor Performance Status Patients with Non-small Cell Lung Cancer", JOURNAL OF KOREAN MEDICAL SCIENCE, vol. 23, no. 6, 1 January 2008 (2008-01-01), page 992, XP055126959, ISSN: 1011-8934, DOI: 10.3346/jkms.2008.23.6.992
- SEIJI NIHO ET AL: "Combination second-line chemotherapy with gemcitabine and docetaxel for recurrent non-small-cell lung cancer after platinum-containing chemotherapy: a phase I/II trial", CANCER CHEMOTHERAPY AND PHARMACOLOGY, vol. 52, no. 1, 1 July 2003 (2003-07-01), pages 19-24, XP055126966, ISSN: 0344-5704, DOI: 10.1007/s00280-003-0618-8
- R. C. DOEBELE ET AL: "A phase I, open-label dose-escalation study of continuous treatment with BIBF 1120 in combination with paclitaxel and carboplatin as first-line treatment in patients with advanced non-small-cell lung cancer", ANNALS OF ONCOLOGY, vol. 23, no. 8, 16 February 2012 (2012-02-16), pages 2094-2102, XP055126867, ISSN: 0923-7534, DOI: 10.1093/annonc/mdr596

## Description

The present invention relates to the field of medicine and especially to the treatment of non-small cell lung cancer (NSCLC), as well as to a clinical indicator (hereinafter designated as clinical marker), useful as predictive variable of the responsiveness of a NSCLC tumour to a treatment. The present invention also relates to a method for designing an individual therapy for a subject suffering from NSCLC as well as to a method for selecting patients likely to respond to a given therapy.

The present invention relates more specifically to an efficacious, and thus beneficial, treatment for a sub-group of tumours in patients suffering from NSCLC, and to a clinical marker useful as predictive variable of the responsiveness of tumours in patients suffering from NSCLC subject to this treatment, the treatment being a treatment with a vascular endothelial growth factor inhibitor or with a vascular endothelial growth factor receptor inhibitor, and more specifically with the compound 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone or a pharmaceutically acceptable salt thereof, and especially its monoethanesulphonate salt form, when used alone or in separate, simultaneous or sequential combination with further pharmaceutically active ingredients and/or further therapeutic treatments.

### Abbreviations

- Alk: anaplastic lymphoma kinase
- bid: administration twice daily
- CI: confidence interval
- CT: computer tomography
- ECOG: Eastern Cooperative Oncology Group
- EGFR: epithelial growth factor receptor
- EMA: European Medicine Agency
- FDA: Food and Drug Administration
- H₀: null hypothesis
- HR: hazard ratio
- INN: International Non-Proprietary Name
- iv: intravenous administration
- NSCLC: non-small cell lung cancer
- OS: overall survival
- p: multiplicity adjusted p-value according to Bonferroni statistical method
- PFS: progression free survival
- po: oral administration
- q21d: administration every 21 day
- VEGF: vascular endothelial growth factor
- VEGFR: vascular endothelial growth factor receptor

### Background to the invention

Routine cancer management using chemotherapy, whether as definitive or adjuvant therapy, has improved patient's absolute survival when compared with non-chemotherapy control. However, not all the chemotherapeutic treatments available are suitable for all patients. The efficacy of chemotherapeutic drugs in patients suffering from cancer may be influenced by several factors, such as for example the presence of certain genetic tumour markers. Other factors may be relevant as well, such as markers of the progression of the disease which are monitored before, during or after treatment. Patients whose tumours have low probability to respond to a chemotherapeutic treatment may omit chemotherapy altogether or may be candidates for alternative treatments, avoiding unnecessary therapeutic side effects.

Therefore, there is a necessity for a personalized approach for the treatment of the disease, particularly in cancers such as lung cancer, colorectal cancer, melanoma, pancreas cancer, prostate cancer, glioblastoma, bladder cancer, ovarian cancer, hepatobiliary cancer, renal cell cancer, breast cancer, head and neck cancer, and lymphomas.

Lung cancer is one of the leading causes of worldwide death. There are two main groups of lung cancers which belong to the lung carcinomas, namely the small cell lung cancers (SCLC) and the non-small cell lung cancers (NSCLC). This classification is currently based on the histology of the tumours, determined by the subcellular details in cells and tissues observed under light microscopy and supplemented by immunohistochemical assays. NSCLC accounts for approximately 70% to 80% of all lung cancers, with 1.2 million new cases diagnosed worldwide each year.

There are three main histology subtypes of NSCLC. The tumour cells in these NSCLC subtypes differ in their size, shape, and immunohistochemical make-up.

Squamous cell or epidermoid carcinoma is a first subtype of NSCLC. About 25% to 30% of all lung cancers are squamous cell carcinomas. These cancers start in early versions of squamous cells, which are flat cells that line the inside of the airways in the lungs. These cancers are often linked to a history of smoking and tend to be found in the middle of the lungs, near a bronchus.

Adenocarcinoma is a second subtype of NSCLC. About 40% of all lung cancers are adenocarcinomas. These cancers start in early versions of the cells that would normally secrete substances such as mucus. This type of lung cancer occurs mainly in people who smoke or have smoked, but it is also the most common type of lung cancer seen in non-smokers. It is more common in women than in men, and it is more likely to occur in younger people than other types of lung cancer. Adenocarcinoma is usually found in the outer region of the lung. It tends to grow faster when compared to squamous cell cancer, and is more likely to be diagnosed before it has spread outside of the lung. This subtype belongs to the non-squamous cell carcinoma.

Large cell carcinoma is a third subtype of NSCLC. About 10% to 20% of all lung cancers are large cell carcinomas. They are named for the appearance of large round cells when examined under the microscope, although the tumors themselves tend to be large as well when diagnosed. Large cell carcinomas often occur in the outer regions of the lungs, and tend to grow rapidly and spread more quickly than some other forms of non-small cell lung cancer. They tend to grow rapidly, metastasize early, and are strongly associated with smoking.

A further subtype of NSCLC is the undifferentiated carcinomas. It comprises the tumours that cannot be identified under the microscope as squamous cell carcinomas, adenocarcinomas or large cell carcinomas. This type of cancer accounts for about 10% to 15% of all lung cancers. It may appear in any part of the lung. It tends to grow and spread quickly, which can make it harder to treat.

In the early stages of disease, lung cancer tends to be asymptomatic. Consequently, at the time of diagnosis, most tumours are overtly (stage IIIB, IV) or covertly metastatic. Resectable, localised, disease (stages I-IIIA) is identified in approximately 20% of patients. Generally advanced stage at diagnosis and the relative resistance of the disease to currently available anti-cancer drugs leads to a high mortality rate, with 5-year survival typically between 10 and 15%.

NSCLC resulted in more than one million annual deaths worldwide in 2001, in more than 300,000 annual deaths in the USA in 2011, and is the leading cause of cancer-related mortality in both men and women (31% and 25%, respectively). The prognosis of advanced NSCLC is dismal.

NSCLC is a difficult to treat disease with a generally short suvival time. Around 80% of the advanced non-squamous NSCLC patients are being treated with a platinuim-containing combination regimen in first line resulting in a median Progression Free Survival PFS of around 7 months and a median Overall Survival OS of around 13 months.

Only a subpopulation of 20% of the non-squamous cell cancer patients harboring tumors with either activating EGFR mutations or the Alk translocation are being treated with a monotherapy of an EGFR- (erlotinib, gefitinib) or Alk-inhibitor (crizotinib) resulting in a median PFS of around 20 months, but thus far no OS improvements have been observed for these patients.

Recently, the maintenance concept has been introduced as one treatment alternative for patients with at least stable disease after 4-6 cycles of 1^{st} line platinum containing combination regimen. Those patients could be treated with pemetrexed, gefitinib or erlotinib in order to maintain tumor stabilization as long as possible.

In contrast, there is no real effective treatment available for patients whose tumor progressed during (refractory tumours) or shortly after the end (resistant tumours) of the 1^{st} line platinum combination regimen or for whom the maintenance concept is not appropriate. These patients are being treated palliatively with docetaxel, pemetrexed or erlotinib after progression has been observed. Ovarian cancer is an accepted disease model for platinum refractory or resistant patients. Patients who progress within 6 months after end of 1^{st} line platinum containing combination regimen are called platinum resistant patients. These patients have a short median PFS and OS in 1^{st} line and their subsequent 2^{nd} line treatment contains no platinum anymore. This treatment principle has however not been suggested for the treatment of non-squamous NSCLC patients, although most of the patients are receiving platinum containing combination therapies in 1^{st} line.

Thus far, only one phase III study has been performed as a post-approval commitment for the FDA and EMA in the segment of 1^{st} line platinum refractory non-squamous NSCLC patients. This multicentre, international, open-label, phase III study, known as TITAN, investigated the efficacy and tolerability of erlotinib versus chemotherapy (docetaxel or pemetrexed) as second-line therapy for NSCLC after fast progression on first-line platinum doublet chemotherapy. However, erlotinib did not show any superior efficacy in PFS and OS as compared to docetaxel or pemetrexed in these fast progressing patients who failed during their 1^{st} line platinum containing therapy.

Overall, there is a high unmet medical need for this population and there are no effective treatments available for these fast progressing refractory or resistant platinum NSCLC patients.

On the other hand, VEGF- or VEGFR-inhibitors showed only modest PFS improvements or failed in first and second line NSCLC patients. Thus far, no reliable clinical markers or other biomarkers have been identified to select patients who are most likely to benefit from a VEGF- or VEGFR-inhibitor treatment and save others from side effects and ineffective treatments.

The compound 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone (INN name nintedanib) is an innovative active ingredient having valuable pharmacological properties, especially for the treatment of oncological diseases, immunologic diseases or pathological conditions involving an immunologic component, or fibrotic diseases.

The chemical structure of this compound is depicted below as Formula A.

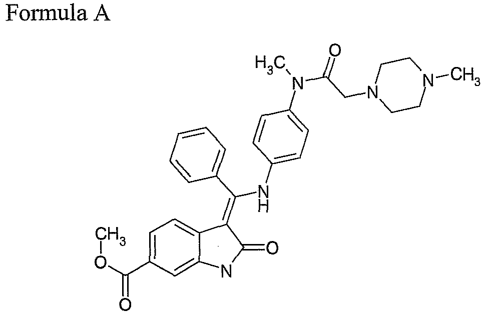

The base form of this compound is described in WO 01/27081, the monoethanesulphonate salt form is described in WO 2004/013099 and various further salt forms are presented in WO 2007/141283. An improved manufacturing process to produce this compound is described in WO 2009/071523 and WO 2009/071524. A capsule pharmaceutical dosage form for immediate release comprising a suspension formulation of this compound is described in WO 2009/147212 and WO 2009/147220.

The monoethanesulphonate salt form of this compound presents properties which makes this salt form especially suitable for development as medicament. The chemical structure of 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone-monoethanesulphonate (INN name nintedanib esylate) is depicted below as Formula A1.

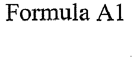

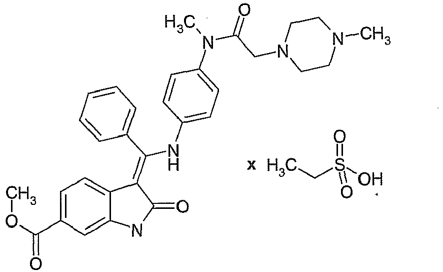

Preclinical studies have shown that this compound is a highly potent, orally bioavailable inhibitor of the vascular endothelial growth factor receptors (VEGFRs), platelet-derived growth factor receptors (PDGFRs), fibroblast growth factor receptors (FGFRs) and of further kinases, that suppress tumor growth through mechanisms inhibiting tumor neovascularization. It has further been shown that this compound inhibits signalling in endothelial- and smooth muscle cells and pericytes, and reduces tumor vessel density.

Furthermore, this compound shows *in vivo* anti-tumor efficacy in all models tested so far at well tolerated doses. The following Table 1 shows the results of the *in vivo* anti-tumor efficacy testing in xenograft models and in a syngeneic rat tumor model.

**Table 1**

| **Cancer** | **Model** | **Efficacy** |
|---|---|---|
| Colorectal | HT-29 | T/C 16% @ 100mg/kg/d |
| | HT-29 large tumors | tumor volume reduction |
| Glioblastoma | GS-9L syngeneic rat | T/C 32% @ 50mg/kg/d |
| Head and neck | FaDu | T/C 11% @ 100mg/kg/d |
| Lung (non-small-cell) | NCI-H460 | T/C 54% @ 25mg/kg/d |
| | Calu-6 | T/C 24% @ 50mg/kg/d |
| Ovarian | SKOV3 | T/C 19% @ 50mg/kg/d |
| Prostate (hormone-dependent) | PAC-120 | T/C 34% @ 100mg/kg/d |
| Renal | Caki-1 | T/C 13% @ 100mg/kg/d |
| Pancreas (murine transgenic) | Rip-Tag | interference with tumor formation |

| | | |
|---|---|---|
| T/C represents the reduction of tumor size in % of the control | | |

This compound is thus for example suitable for the treatment of diseases in which angiogenesis or the proliferation of cells is involved. The use of this compound for the treatment of immunologic diseases or pathological conditions involving an immunologic component is being described in WO 2004/017948, the use for the treatment of, amongst others, oncological diseases, alone or in combination, is being described in WO 2004/096224 and WO 2009/147218, and the use for the treatment of fibrotic. diseases is being described in WO 2006/067165.

A method using biomarkers for monitoring the treatment of an individual with the compound 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone or a pharmaceutically acceptable salt thereof, wherein it is determined if a sample from said individual comprises a biomarker in an amount that is indicative for said treatment, is disclosed in WO 2010/103058.

### Summary of the invention

The inventors of the present invention have discovered that nintedanib can be used in a method, or for the preparation of a medicament, for the treatment of non-small cell lung cancer (NSCLC) in a patient who has received prior treatment with an anti-tumour therapy other than with nintedanib, which method comprises treating said patient with a treatment regimen comprising the administration of nintedanib or a pharmaceutically acceptable salt thereof, wherein the patient to be treated is selected for treatment on the basis of having shown progression of the cancer within a period of 9 months or less after the initiation of said prior treatment.

The inventors of the present invention have further discovered that a clinical trial evidenced improvement in the median of both Progression Free Survival (PFS) and Overall Survival (OS) superior or equal to one month when compared to placebo was obtained when the patient to be treated is selected for treatment on the basis of having shown progression of the cancer within a period of 9 months or less after the initiation of said prior treatment. These patients are the refractory and resistant patients having shown progression of the cancer within a period of 9 months or less after the initiation of said prior treatment.

In one embodiement in accordance with the present invention, the histology of the tumour belongs to the subtype of the squamous cell or epidermoid carcinoma NSCLC tumours.

In a further embodiment in accordance with the present invention, the histology of the tumour belongs to the subtype of the adenocarcinoma NSCLC tumours.

In a further embodiment in accordance with the present invention, the histology of the tumour belongs to the subtype of the large cell carcinoma NSCLC tumours.

In a further embodiment in accordance with the present invention, the histology of the tumour belongs to the subtype of the undifferentiated carcinoma NSCLC tumours.

The inventors of the present invention have further discovered that a clinical trial evidenced improvement in the median of both Progression Free Survival (PFS) and Overall Survival (OS) superior or equal to 1.3 months when compared to placebo was obtained with nintedanib when the histology of the tumour belongs to the subtype of the adenocarcinoma NSCLC tumours and when the patient to be treated is selected for treatment on the basis of having shown progression of the cancer within a period of 9 months or less after the initiation of said prior treatment. These patients are the refractory and resistant patients having shown progression of the cancer within a period of 9 months or less after the initiation of said prior treatment with an anti-tumour therapy other than with nintedanib.

In a further embodiment in accordance with the present invention, the prior treatment with an anti-tumour therapy other than with nintedanib is a monotherapeutic treatment with a platinum compound, or a combination treatment of a platinum compound with one or more further therapeutic agents other than nintedanib, with or without adjuvant or neoadjuvant therapy.

In a further embodiment in accordance with the present invention, the treatment regimen comprising the administration of nintedanib or a pharmaceutically acceptable salt thereof is a treatment regimen with the compound 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone (INN name nintedanib) or a pharmaceutically acceptable salt thereof, and especially its monoethanesulphonate salt (INN name nintedanib esylate), when used alone or in separate, simultaneous or sequential combination with further pharmaceutically active ingredients and/or further therapeutic treatments.

In a further embodiment, the separate, simultaneous or sequential combination of nintedanib or a pharmaceutically acceptable salt thereof with further pharmaceutically active ingredients and/or further treatments is with an anti-cancer drug from plants, with an anti-folate, or with an EGFR inhibitor.

In a further embodiment in accordance with the present invention, the treatment regimen comprising the administration of nintedanib or a pharmaceutically acceptable salt thereof is a combination treatment regimen with an anti-cancer drug from plants selected from docetaxel or paclitaxel, or a pharmaceutically acceptable salt thereof, or with an anti-folate selected from pemetrexed or pralatrexate, or a pharmaceutically acceptable salt thereof, or with an EGFR inhibitor such as erlotinib, gefitinib or afatinib. Especially preferred anti-cancer drug from plants is docetaxel, or a pharmaceutically acceptable salt thereof. Especially preferred anti-folate is pemetrexed, or a pharmaceutically acceptable salt thereof.

The inventors of the present invention have further discovered that a time period of less than 9 months until progression of the tumour since initiation of a treatment with an anti-tumour therapy other than with nintedanib delimits the group of patients with fast refractory and resistant tumours to this treatment which benefit from a clinically effective treatment with nintedanib or a pharmaceutically acceptable salt thereof in accordance with the present invention, and thus delimits the patient population, which, in accordance with the present invention, benefits from this clinically effective treatment with nintedanib or a pharmaceutically acceptable salt thereof. In other words, this time period of less than 9 months until progression since initiation of a treatment with an anti-tumour therapy other than with nintedanib is a predictive clinical marker for a treatment of NSCLC patients to be treated with nintedanib or a pharmaceutically acceptable salt thereof in accordance with the present invention.

A predictive clinical marker provides information as to the chances to obtain a therapeutic effect from a treatment with a particular drug, which, in the case of the present invention, is nintedanib or a pharmaceutically acceptable salt thereof.

In a further embodiment, a time period of between 4 and 9 months until progression since initiation of a prior treatment with an anti-tumour therapy other than with nintedanib is a predictive clinical marker for a treatment of NSCLC patients to be treated with nintedanib or a pharmaceutically acceptable salt thereof in accordance with the present invention.

In a further embodiment, a time period of 4, 5, 6, 7, 8 or 9 months until progression since initiation of a prior treatment with an anti-tumour therapy other than with nintedanib is a predictive clinical marker for a treatment of NSCLC patients to be treated with nintedanib or a pharmaceutically acceptable salt thereof in accordance with the present invention.

In a further embodiment, a time period of less than 4 months until progression since initiation of a prior treatment with an anti-tumour therapy other than with nintedanib is a predictive clinical marker for a treatment of NSCLC patients to be treated with nintedanib or a pharmaceutically acceptable salt thereof in accordance with the present invention.

One further object of the present invention is thus to provide the hereinbefore described clinical marker of the responsiveness of a subject suffering from a NSCLC tumour to a treatment with nintedanib or a pharmaceutically acceptable salt thereof.

A further object of the present invention is to provide a method for designing an individual therapy for a subject suffering from a NSCLC tumour, wherein said method comprises the use of the hereinbefore described clinical marker.

A further object of the present invention is to provide a method for selecting patients likely to respond to a given therapy, wherein said method comprises the use of the hereinbefore described clinical marker.

A further object of the present invention is to provide a method for the efficacious treatment for the refractory and for the resistant NSCLC tumours in a patient, wherein said method comprises the use of the hereinbefore described clinical marker.

A further object of the present invention is to provide a method for delaying disease progression and prolonging patient survival of a patient suffering from a NSCLC tumour, wherein said method comprises the use of the hereinbefore described clinical marker.

In a further embodiment, the prior treatment with an anti-tumour therapy other than with nintedanib is a treatment with a platinum compound, such as carboplatin, cisplatin, or oxaliplatin, or a pharmaceutically acceptable salt thereof. Especially preferred platinum compound to be used in a prior treatment in accordance with the present uinvention is carboplatin or cisplatin. The prior treatment with an anti-tumour therapy other than with nintedanib may also be a combination treatment of a platinum compound with a further chemotherapeutic agent, such as paclitaxel, gemcitabine, a vinka alkaloid or etoposide, or a pharmaceutically acceptable salt thereof, or a VEGF inhibitor such as bevacizumab. If this treatment is the first anti-tumour therapy for this patient and is performed with a platinum compound, it is typically called a "1^{st} line platinum based treatment or therapy". By definition (online definition provided by the National Cancer Institute at the National Institutes of Health), a 1^{st} line therapy is the first treatment given for a disease. It is often part of a standard set of treatments, such as surgery followed by chemotherapy and radiation. When used by itself, the 1^{st} line therapy is the one accepted as the best treatment. If it doesn't cure the disease or cause severe side effects, other treatment may be added or used instead. It is also called induction therapy, primary therapy, and primary treatment.

The next anti-tumour therapy for this patient will then typically be called 2^{nd} line therapy. By definition (online definition provided by the National Cancer Institute at the National Institutes of Health), a 2^{nd} line therapy is a treatment that is given when initial treatment (or 1^{st} line therapy) doesn't work, or stops working.

Hence, in accordance with the present invention, previous cancer treatments may have been surgery and/or radiotherapy, with or without adjuvant or neoadjuvant therapy.

### Legend to the Figures

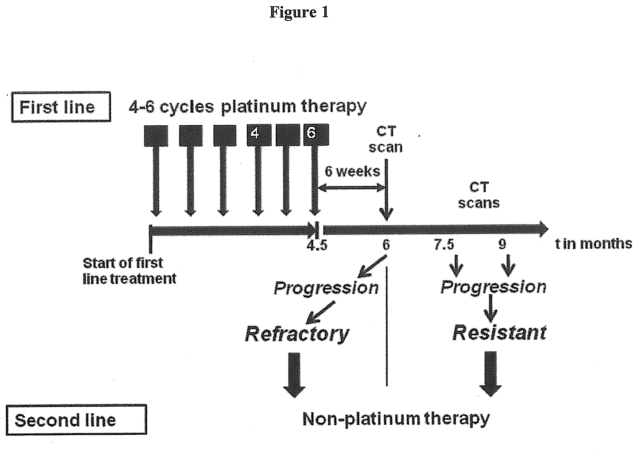

Figure 1: Graphical representation of a typical 1^{st} line platinum based therapy of NSCLC tumours (6 cycles of therapy representing 4.5 months of treatment duration) and of a 2^{nd} line non-platinum based therapy of fast progressing refractory (identified via computer tomoraphy CT within 6 months after the start of 1^{st} line platinum based therapy) and resistant (identified via computer tomography CT after the 6 months following 1^{st} line platinum based therapy) NSCLC tumours.
   In the following Figures (Kaplan-Meier plots), N is the number of patients in each treatment arm, the ordinate of the graph (%) represents the estimated percentage of PFS or OS for the specific patient population analyzed, and the abscissa of the graph (T) represents the time from randomization in 2^{nd} line treatment in months.

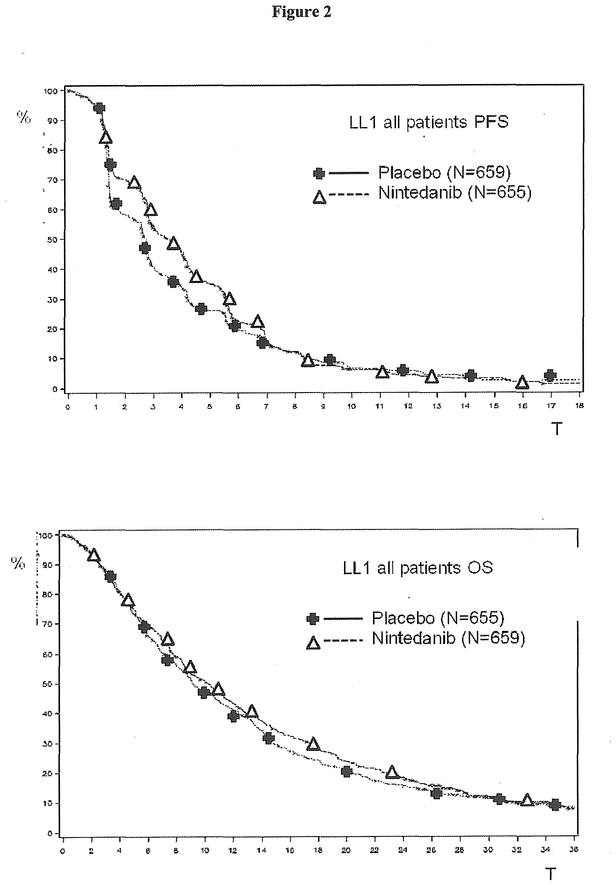

Figure 2: The top graphic shows the result of the PFS independent review of the whole population of NSCLC patients (LUME LUNG 1 study). The bottom graphic shows the OS results of the whole population of NSCLC patients (LUME LUNG 1 study).

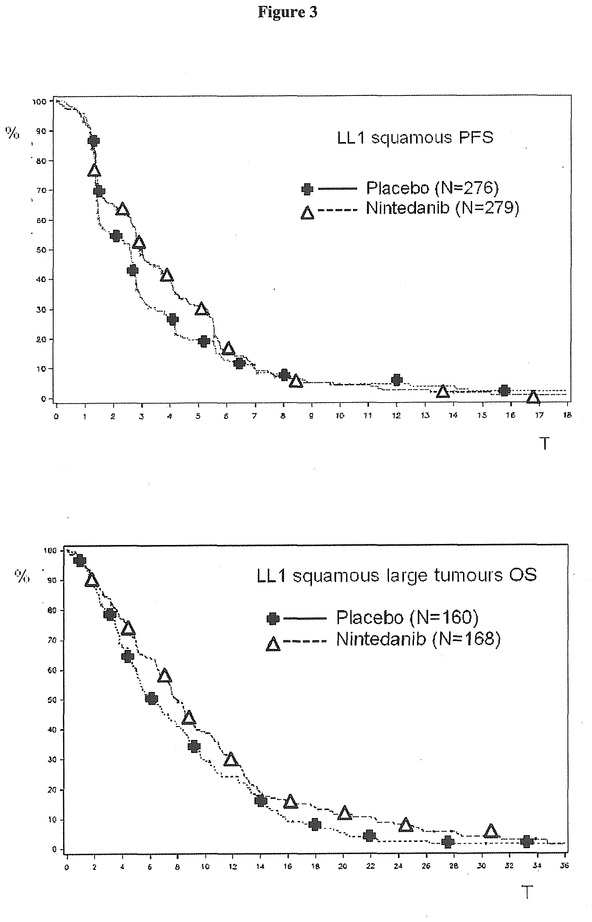

Figure 3: The top graphic shows the result of the PFS independent review of the target population of squamous cell tumour histology NSCLC patients (LUME LUNG 1 study). The bottom graphic shows the OS results of the target population of squamous cell cancer histology with large tumour burden of NSCLC patients (LUME LUNG 1 study).

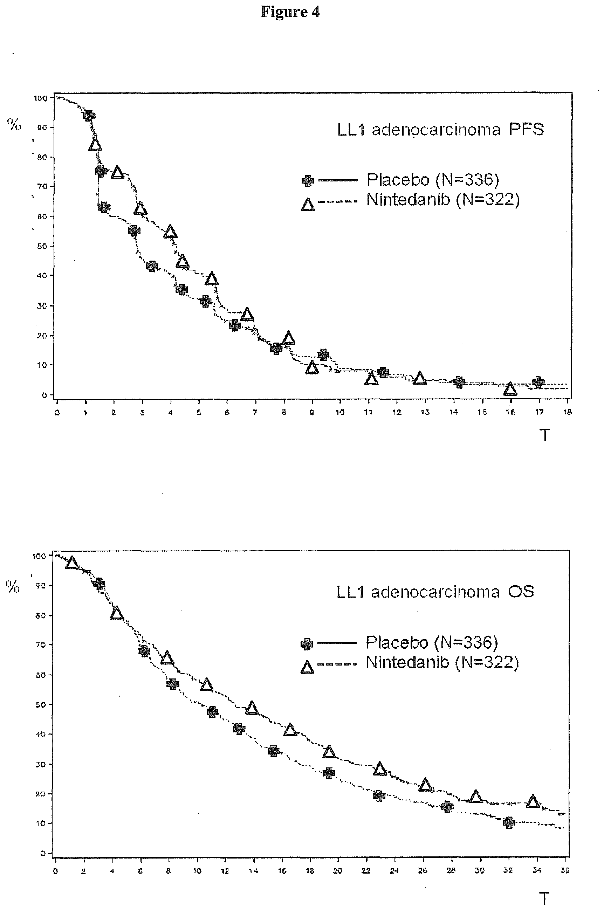

Figure 4: The top graphic shows the result of the PFS independent review of the target population of adenocarcinoma tumour histology NSCLC patients (LUME LUNG 1 study). The bottom graphic shows the OS results of the target population of adenocarcinoma tumour histology NSCLC patients (LUME LUNG 1 study).

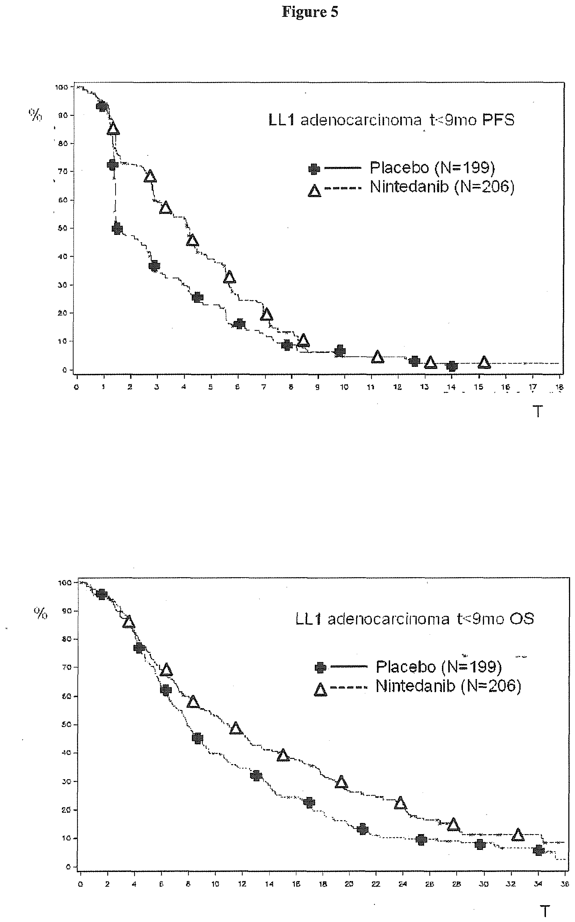

Figure 5: The top graphic shows the result of the PFS independent review of the target population of refractory and resistant adenocarcinoma tumour NSCLC patients with a time t since start of first line therapy inferior 9 months (LUME LUNG 1 study). The bottom graphic shows the OS results of the target population of refractory and resistant adenocarcinoma tumour NSCLC patients, with a time since start of first line therapy inferior 9 months (LUME LUNG 1 study).

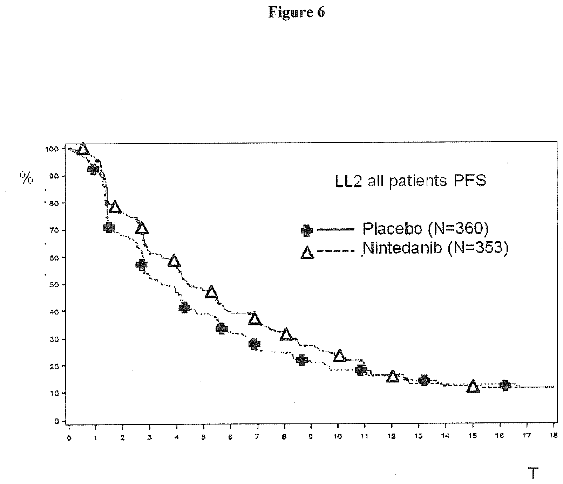

Figure 6: The graphic shows the result of the PFS independent review of the target population of adenocarcinoma tumour histology NSCLC patients (LUME LUNG 2 study).

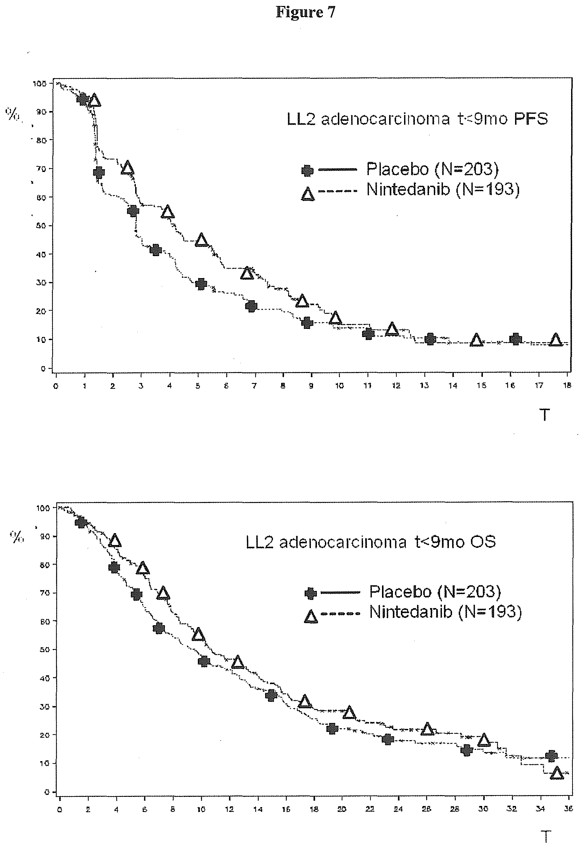

Figure 7: The top graphic shows the result of the PFS independent review of the target population of refractory and resistant adenocarcinoma tumour NSCLC patients with a time t since start of first line therapy inferior 9 months (LUME LUNG 2 study). The bottom graphic shows the OS results of the target population of refractory and resistant adenocarcinoma tumour NSCLC patients, with a time since start of first line therapy inferior 9 months (LUME LUNG 2 study).

### Detailed description of the invention

Within the scope of the present invention, an efficacious treatment for NSCLC patients is provided.

Further withtin the scope of the present invention, an efficacious treatment for NSCLC patients with the following tumour histology is provided: the squamous cell carcinoma tumours, the adenocarcinoma tumours, the large cell carcinoma tumours and the undifferentiated carcinoma tumours.

Further within the scope of the present invention, an efficacious treatment for a subpopulation of the NSCLC patients, namely the patients for which the tumours are progressing within a period of 9 months or less after the initiation of a prior treatment with an anti-tumour therapy other than with nintedanib, is provided.

As already mentioned hereinbefore, VEGF- or VEGFR-inhibitors showed only modest PFS improvements or failed in first and second line NSCLC patients. Thus far, no reliable clinical markers or other biomarkers have been identified to select patients who are most likely to benefit from a VEGF- or VEGFR-inhibitor treatment and save others from side effects and ineffective treatments. For NSCLC patients with a very poor prognostic outcome after a first treatment with an anti-tumour therapy other than with nintedanib and a high medical need, the time period until progression since initiation of said prior treatment has been discovered as a predictive clinical marker for a treatment with nintedanib or a pharmaceutically acceptable salt thereof. As predictive clinical marker, this time can be used as a variable to indicate for which patient population a beneficial therapeutic effect can be expected from a treatment with nintedanib, or a pharmaceutical salt thereof, when used alone or in separate, simultaneous or sequential combination with further pharmaceutically active ingredients and/or further treatments.

Within the meaning of the present invention, the time period t until progression since initiation of the prior treatment means the length of time between the start of this treatment (the day on which the patient receive the first administration of this treatment) and the diagnosis of the progression of the tumour (the day on which the tumour of this patient is diagnosed as progressive) or, in case of a clinical study, the day of randomization in the following treatment.

Within the meaning of the present invention, a refractory tumour is defined as a tumour progressing during the time between the prior or 1^{st} line treatment and up to 6 weeks after end of this treatment, i.e. when the first diagnosis to assess if progression occurs is performed.

Within the meaning of the present invention, a resistent tumour is defined as a tumour progressing between 6 weeks after end of the prior or 1^{st} line treatment, i.e. when the first diagnosis to assess if progression occurs is performed, and until 9 months after the start of the prior or 1^{st} line treatment.

Figure 1 is a graphical representation of a typical 1^{st} line platinum based therapy of NSCLC tumours (6 cycles of therapy representing 4.5 months of treatment duration) and of a 2^{nd} line non-platinum based therapy of fast progressing refractory (identified via computer tomoraphy CT within 6 months after the start of 1^{st} line platinum based therapy) and resistant (identified via computer tomography CT after the 6 months following 1^{st} line platinum based therapy) NSCLC tumours.

Within the meaning of the present invention, the progression of a tumour is diagnosed via the following techniques: radiography of the brain and of the chest, including the liver and adrenals, and bone scan in case of bone metastases, performed for example via computer tomography scanning (CT scan). The RECIST criteria for disease progression are used to assess the progression of the tumour, as in the majority of clinical trials evaluating cancer treatments. These criteria, disclosed for example in Eisenhauer EA et al. (New response evaluation criteria in solid tumours: Revised RECIST guideline, version 1.1, Eur J Cancer 2009; 45: 228-247), define that a tumour progresses when there is at least a 20% increase in the sum of diameters of target lesions, taking as reference the smallest sum on study (this includes the baseline sum if that is the smallest on study). In addition to the relative increase of 20%, the sum must also demonstrate an absolute increase of at least 5 mm. The appearance of one or more new lesions is also considered as progression.

Within the meaning of the present invention, Progression Free Survival (PFS) is the time between the day of randomization in a clinical study and the diagnosis of the progression of the tumour or death of the patient, whichever occurs earlier. This definition is also the definition given by the US Food and Drug Administration for PFS.

Within the meaning of the present invention, Overall Survival (OS) is the time between the day of randomization in a clinical study and the death of the patient for any cause. This definition is also the definition given by the US Food and Drug Administration for OS.

The following details and results concerning the phase III clinical studies LUME LUNG 1 and 2 are intended to illustrate the present invention.

Nintedanib, a triple angiokinase inhibitor, is in advanced development with two pivotal trials in 2^{nd} line NSCLC. The clinical study LUME LUNG 1 investigates the combination of nintedanib and docetaxel versus docetaxel and placebo in second line advanced NSCLC patients. The clinical study LUME LUNG 2 investigates the combination of nintedanib and pemetrexed versus pemetrexed and placebo in second line advanced NSCLC patients.

For the clinical studies, the active ingredients are administered in the form of their respective pharmaceutically acceptable salts. In the case of nintedanib, this is the salt form as used in development and during the clinical studies performed for marketing authorization, namely the monoethanseulfonate salt form of the compound (INN name nintedanib esylate). In the case of pemetrexed, this is the salt form as can be found on the market, namely the disodium salt form.

In the studies described in the foregoing, a 1^{st} line treatment includes a limited number of cytotoxic chemotherapy, targeted therapy or maintenance treatment after end of cytotoxic chemotherapy.

### Phase 3 clinical study LUME LUNG 1 (LL1): a multicenter, randomized, double-blind clinical trial

### Background

LUME LUNG 1 is a placebo-controlled phase 3 trial investigating nintedanib + docetaxel in patients with locally advanced/metastatic NSCLC progressing after one prior combination regimen. LUME LUNG 1 randomized 1314 patients (all histologies) to nintedanib + docetaxel or placebo + docetaxel.

### Method

Eligible stage IIIB/IV NSCLC patients (stratified by ECOG performance status, prior bevacizumab treatment, histology of the tumour and presence of brain metastases) were randomized 1:1 to nintedanib 200 mg po bid + docetaxel 75 mg/m² iv q21d (N=655; Arm A) or to placebo + docetaxel (N=659; Arm B). H₀ was tested on the primary endpoint of centrally reviewed PFS after 713 events (two-sided α=5%, β=10%). The secondary endpoint of OS was tested hierarchically (overall α=5% two sided, β=20%), first in adenocarcinoma patients by time t since start of 1^{st} line therapy inferior to 9 months (t<9mo), followed by all adenocarcinoma diagnosed patients, and then all patients. This hierarchy was introduced after explanatory analyses identified t<9mo as a predictive clinical marker for nintedanib treatment prior to unblinding for the final OS analysis.

### Results

Patient characteristics were balanced between the two arms.

A benefit for nintedanib versus placebo was seen in the whole NSCLC patient population of this study, with a median improvement of centrally assessed PFS of 0.8 months in nintedanib treated patients versus placebo (median PFS of 3.5 months for nintedanib treated patients in Arm A versus 2.7 months for placebo in Arm B), and with a median improvement of OS of 1 month in nintedanib treated patients versus placebo (median PFS of 10.1 months for nintedanib treated patients in Arm A versus 9.1 months for placebo in Arm B). The result is shown in Figure 2 and Table 2.

A benefit for nintedanib versus placebo was seen in the patient population of this study with NSCLC squamous cell tumour histology, with a median improvement of PFS of 0.4 months in nintedanib treated patients versus placebo (median PFS of 3.0 months for nintedanib + docetaxel treated patients in Arm A versus 2.6 months for placebo + docetaxel treated patients in Arm B). The result is shown in Figure 3 and Table 2.

No benefit was shown in OS survival between nintedanib treated patients versus placebo for the patients with NSCLC tumours of squamous cell histology..

However, a benefit for nintedanib versus placebo was seen in the patient population of this study with NSCLC squamous cell tumour histology and large tumour burden, with a median improvement of OS of 1.6 months in nintedanib treated patients versus placebo (median OS of 7.7 months for nintedanib + docetaxel treated patients in Arm A versus 6.1 months for placebo + docetaxel treated patients in Arm B). The result is shown in Figure 3 and Table 2.

A benefit for nintedanib versus placebo was seen in the patient population of this study with NSCLC adenocarcinoma tumour histology, with a median improvement of PFS of 1.4 months in nintedanib treated patients versus placebo (median PFS of 4.2 months for nintedanib + docetaxel treated patients in Arm A versus 2.8 months for placebo + docetaxel treated patients in Arm B), and with a median improvement of OS of 2.3 months in nintedanib treated patients versus placebo (median PFS of 12.6 months for nintedanib treated patients in Arm A versus 10.3 months for placebo in Arm B). The result is shown in Figure 4 and Table 2.

Interaction tests and HR 95% CI were used to select a cut-off of 9 months since start of 1^{st} line therapy to define the fast progressing target population (t<9mo).

A benefit for nintedanib versus placebo was further seen in the fast progressing t<9mo patient population of this study with NSCLC adenocarcinoma tumour histology, with a median improvement of PFS of 2.7 months in nintedanib treated patients versus placebo (median PFS of 4.2 months for nintedanib + docetaxel treated patients versus 1.5 months for placebo + docetaxel treated patients, with an HR of 0.68 and a CI of 0.54-0.84, and with p=0.0005), and with a median improvement of OS of 3 months in nintedanib treated patients versus placebo (median PFS of 10.9 months for nintedanib treated patients in Arm A versus 7.9 months for placebo in Arm B). The result is shown in Figure 5 and Table 2.

Above benefit has also been confirmed in the fast progressing NSCLC patient population selected with a cut-off of 4 or 6 months since start of 1^{st} line therapy of this study with NSCLC adenocarcinoma tumour histology (t<4mo or t<6mo fast progressing target population).

### Conclusion

The LUME LUNG 1 study thus met its primary endpoint of PFS. The LUME LUNG 1 study thus met also its secondary endpoint of OS. Further detailed data and results will be presented at the American Society of Clinical Oncology (ASCO) Congress in 2013. Time since start of 1^{st} line treatment was the only prognostic and predictive clinical marker for 2^{nd} line nintedanib combination treatment in adavnced NSCLC patients. A time t inferior 9 months was validated in this NSCLC trial as a predictive clinical marker of nintedanib 2^{nd} line treatment benefit for the NSCLC patients most refractory or resistant to platinum-based 1^{st} line therapy.

It can be expected that above benefit is confirmed in the fast progressing NSCLC patient populations of other histology subtypes than adenocarcinoma histology, such as the squamous cell carcinomas, the large cell carcinomas and the undifferentiated carcinomas. It can also be expected that above benefit is confirmed in the fast progressing NSCLC patient populations of the subtypes squamous cell carcinoma, large cell carcinoma and undifferentiated carcinoma, selected with a cut-off of less than 9 months since start of 1^{st} line therapy, such as for example a cut-off of 4 or 6 months since start of 1^{st} line therapy (t<4mo or t<6mo fast progressing target population).

### Phase 3 clinical study LUME LUNG 2 (LL2): a multicenter, randomized, double-blind clinical trial

### Background

LUME LUNG 2 is a placebo-controlled phase 3 trial investigating the safety and efficacy of nintedanib + pemetrexed versus placebo + pemetrexed in patients with advanced NSCLC tumours previously treated with chemotherapy. LUME LUNG 2 randomized patients to nintedanib + pemetrexed or placebo + pemetrexed.

### Method

Eligible stage IIIB/IV NSCLC patients (stratified by ECOG performance status, prior bevacizumab treatment, histology of the tumour and presence of brain metastases) were randomized 1:1 to nintedanib 200 mg po bid + pemetrexed 500 mg/m² iv q21d (n=351, Arm A) or placebo + pemetrexed 500 mg/m² iv q21d (n=349, Arm B). Continuation until Progressive Disease (PD) or unacceptable toxicity with nintedanib, placebo, pemetrexed, or a combination thereof, was permitted. H₀ was tested on the Intention To Treat (ITT) population after 394 events had occurred (two sided α=5%). Primary endpoint was centrally assessed PFS. Secondary endpoints included OS, investigator-assessed PFS, Response Rate (RR), safety, and Quality of Life (QoL). Stepwise selection, using Cox proportional hazards modeling and a recursive partitioning tree, identified baseline variables prognostic for PFS in Arm B (placebo) of LUME LUNG 2. Covariate interaction tests and Hazard Ratio (HR) interaction plots showed factors predictive of improved PFS in Arm A (nintedanib treated patients) of LUME LUNG 2. These methods were applied to the LUME LUNG 2 Data Monitoring Committee (DMC) dataset, to develop a hypothesis that was tested and validated by three steps: (i) internal confirmation using centrally assessed PFS and internal validation using OS data of the clinical trial LL2; (ii) external validation using the data from the primary analysis of the independent clinical trial LL1 (centrally assessed PFS and internal validation using OS data); and (iii) external validation using the final OS data of the clinical trial LL1.

### Results

Baseline patient characteristics were balanced between nintedanib + pemetrexed (Arm A) versus placebo + pemetrexed (Arm B): median age was 59 years in both patient populations; frequency of female patients was 45% (Arm A) versus 42% (Arm B); frequency of ECOG performance status I patients was 62% (Arm A) versus 61%(Arm B); frequency of adenocarcinoma patients was 95% (Arm A) versus 93% (Arm A); prior treatment with bevacizumab was 8% in both patient populations.

Based on a planned Data Monitoring Committee (DMC) futility analysis of investigator-assessed PFS, enrolment was halted after randomizing 713/1300 planned patients. However, no safety issues were identified. Ongoing patients were unblinded and follow-up was continued in accordance with the clinical trial protocol. Subsequent Intention To Treat (ITT) analysis of the primary endpoint (centrally reviewed PFS) favored nintedanib treated patients (Arm A) versus placebo (Arm B), with a median PFS of 4.4 for nintedanib treated patients (Arm A) versus 3.4 months for placebo (Arm B). Thus, a median improvement of PFS of 1 month in nintedanib treated patients versus placebo was seen. Disease control was also significantly improved in nintedanib treated patients. The result is shown in Figure 6 and Table 2. No difference in Overall Survival (HR 1.01) was however found between both arms.

Time t since start of 1^{st} line was the only prognostic and predictive clinical marker for 2^{nd} line nintedanib combination in adenocarcinoma diagnosed NSCLC patients. Interaction tests and Hazard Ratio (HR) 95% Confidence Intervals (CI) were used to select a cut-off of 9 months since start of 1^{st} line to define the target population (t<9mo). A benefit of 1.3 months for nintedanib treated patients versus placebo in the t<9mo patient population was seen in LUME LUNG 2, with a median PFS for nintedanib treated patients (Arm A) of 4.1 months versus 2.8 months for placebo (Arm B). furthermore, a benefit of 1.3 months for nintedanib treated patients versus placebo in the t<9mo patient population was seen in LUME LUNG 2, with a median OS for nintedanib treated patients (Arm A) of 10.6 months versus 9.3 months for placebo (Arm B).The result is shown in Figure 7 and Table 2.

### Conclusion

The primary endpoint was met even though the study was stopped prematurely. Treatment with nintedanib + pemetrexed significantly improved centrally reviewed Progression Free Survival (PFS) vs placebo + pemetrexed in patients with advanced NSCLC tumours previously treated with chemotherapy, and had a manageable safety profile. Further detailed data and results will be presented at the American Society of Clinical Oncology (ASCO) Congress in 2013.

In line with the findings of LUME LUNG 1, in LUME LUNG 2, nintedanib in combination with pemetrexed further improved independently assessed PFS in the platinum refractory adenocarcinoma patients (median PFS 4.0 months) as compared to pemetrexed in combination with placebo (median PFS 2.8 months).

A time t< 9 months was validated in this NSCLC trial as a prognostic clinical marker and a predictive clinical marker of nintedanib benefit for the adenocarcinoma patients most refractory to platinum-based 1^{st} line therapy.

Moreover, those patients showed also an improvement in one measure of patient's quality of life. Time to deterioration of cough was significantly prolonged for these fast progressing patients, with a median improvement of time to deterioration of cough of 3.7 months in nintedanib treated patients versus placebo (median time to deterioration of cough of 7.2 months for nintedanib + pemetrexed treated patients versus 3.5 months for placebo + pemetrexed treated patients with a HR of 0.66 (CI: 0.47-0.93, p= 0.0158).

The above described clinical marker effect was more pronounced for patients with adenocarcinoma histology in the independent clinical study LUME LUNG 1 (external validation based on centrally assessed PFS and internal validation using OS data).

These findings have been confirmed as a pre-specified subgroup with the final overall survival (OS) data of the clinical study LUME LUNG 1 (external validation using the final OS data).

It can be expected that above benefit is confirmed in the fast progressing NSCLC patient populations of other histology subtypes than adenocarcinoma histology, such as the squamous cell carcinomas, the large cell carcinomas and the undifferentiated carcinomas. It can also be expected that above benefit is confirmed in the fast progressing NSCLC patient populations of the subtypes squamous cell carcinoma, large cell carcinoma and undifferentiated carcinoma, selected with a cut-off of less than 9 months since start of 1^{st} line therapy, such as for example a cut-off of 4 or 6 months since start of 1^{st} line therapy (t<4mo or t<6mo fast progressing target population).

The results of the statistical analysis of the two clinical trials LUME LUNG 1 (LL1) and LUME LUNG 2 (LL2) are summarized in the Table 2 below.

**Table 2**

| **NSCLC patient population** | **Improvement of median PFS and median OS of nintedanib versus placebo** | **Statistical values** | | |
|---|---|---|---|---|
| | | **HR** | **95% CI** | **p** |
| LL1 all (Fig. 2) | 0.8 months (PFS) | 0.85 | 0.75-0.96 | 0.0070 |
| | 1 month (OS) | 0.94 | 0.83-1.05 | 0.2720 |
| LL1 squamous cell (Fig. 3) | 0.4 months(PFS) | 0.83 | 0.69-1.01 | 0.0569 |
| | None (OS) | 1.01 | 0.85-1.21 | 0.8907 |
| LL1 squamous cell large tumour burden (Fig. 3) | 0.9 months (PFS) | 0.71 | 0.56-0.91 | 0.0072 |
| | 1.6 months (OS) | 0.82 | 0.65-1.04 | 0.0995 |
| LL1 adenocarcinoma (Fig. 4) | 1.4 months (PFS) | 0.84 | 0.71-1.00 | 0.0485 |
| | 2.3 months (OS) | 0.83 | 0.70-0.99 | 0.0359 |
| LL1 adenocarcinoma t<9mo (Fig. 5) | 2.7 months (PFS) | 0.68 | 0.54-0.84 | 0.0005 |
| | 3 months (OS) | 0.75 | 0.60-0.92 | 0.0073 |
| LL2 all (Fig. 6) | 1 months (PFS) | 0.84 | 0.70-1.00 | 0.0506 |
| | None (OS) | 1.01 | 0.85-1.21 | 0.8940 |
| LL2 adenocarcinoma t<9mo (Fig. 7) | 1.3 months (PFS) | 0.79 | 0.63-1.00 | 0.0527 |
| | 1.3 months (OS) | 0.87 | 0.69-1.10 | 0.255 |

| | | | | |
|---|---|---|---|---|
| NSCLC, PFS, OS, HR, CI and p are defined in the Abbreviation section | | | | |

### Statistical methods

Two statistical methods were used for the identification of the subgroups within the clinical trials LUME LUNG 1 and 2, namely the recursive partitioning method developed by Hothorn et al. (Hothorn T et al., Unbiased Recursive Partitioning: A Conditional Inference Framework, Journal of Computational and Graphical Statistics, 2006, Volume 15, Number 3, Pages 651-674), including the permutation test developed by Strasser and Weber (Helmut Strasser & Christian Weber, On the asymptotic theory of permutation statistics, Mathematical Methods of Statistics, 1999, Vol 8, 220-250) and the stepwise selection approach as explained by Collett (D. Collett, Modelling Survival Data in Medical Research, Chapman & Hall/CRC, London, UK, 2nd edition, 2003). In order to investigate whether the identified prognostic factors are also associated with the response to treatment with nintedanib, a treatment-by-covariate interaction test was applied as explained by Collett (D. Collett, Modelling Survival Data in Medical Research, Chapman & Hall/CRC, London, UK, 2nd edition, 2003).

In the case of a significant interaction between treatment and a continuous variable, not only the categorisation delivered by the recursive partitioning was investigated but hazard ratios HR (treatment effect) were also globally estimated at different values of the interacting covariate. Plots of this hazard ratio by treatment interaction and the corresponding 95% CI limits were provided in order to graphically display the dependence of the treatment effect on the interacting covariate.

The covariate values below (or above) for which the HR point estimate is < 1 and for which the width of the 95% CI is small were considered to define a subgroup of patients with greater treatment benefit. Kaplan-Meier plots and Forrest Plots were further used for the graphical presentation of the results.

### Further embodiments

The diseases which may be treated with the the compound 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone or a pharmaceutically acceptable salt thereof, and especially its monoethanesulphonate salt form, when used alone or optionally in combination with further pharmaceutically active ingredients and/or further treatments, such as for example radiotherapy, are diseases involving cell proliferation, migration or apoptosis of myeloma cells, angiogenesis or fibrosis.

In a preferred embodiment, the disease comprises the presence of a tumor.

In a further embodiment, the disease is a progressive tumor. More specifically, the disease is selected from non small cell lung cancer (NSCLC).

As stated above the treatments of the present invention as defined herein are of interest for their antiangiogenic and/or vascular permeability effects. Angiogenesis and/or an increase in vascular permeability is present in a wide range of disease states including cancer (including leukaemia, Kaposi's sarcoma, multiple myeloma lymphoma, NSCLC, mesothelioma, renal cell cancer, hepatocellular cancer, colorectal cancer, ovarian cancer), diabetes, psoriasis, rheumatoid arthritis, haemangioma, acute and chronic nephropathies, atheroma, arterial restenosis, autoimmune diseases, acute inflammation, asthma, lymphoedema, endometriosis, dysfunctional uterine bleeding, fibrosis, cirrhosis and ocular diseases with retinal vessel proliferation including age-related macular degeneration.

A combination treatment of the present invention as defined herein may be achieved by way of the simultaneous, sequential or separate administration of the individual components of said treatment. A combination treatment as defined herein may be applied as a sole therapy or may involve surgery or radiotherapy or an additional chemotherapeutic or targeted agent in addition to a combination treatment of the invention. Surgery may comprise the step of partial or complete tumour resection, prior to, during or after the administration of the combination treatment as described herein.

Treatment with chemotherapeutic agents such as pemetrexed or nintedanib may be associated with adverse events such as myelosuppression and gastrointestinal adverse events, including mucositis, diarrhoea, nausea /vomiting and neutropenia. To prevent these toxicities, patients may receive adequate premedication and concomitant medication with dexamethasone (or an equivalent corticosteroid regimen), folic acid, vitamin B12, loperamide, metoclopramide, prochlorpramide, or dimenhydrinate.

Further pharmaceutically acceptable salts of the compound 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone than those already described hereinbefore may, for example, include acid addition salts. Such acid addition salts include, for example, salts with inorganic or organic acids affording pharmaceutically acceptable anions such as with hydrogen halides or with sulphuric or phosphoric acid, or with trifluoroacetic, citric or maleic acid. In addition, pharmaceutically acceptable salts may be formed with an inorganic or organic base which affords a pharmaceutically acceptable cation. Such salts with inorganic or organic bases include for example alkali metal salts, such as the sodium or potassium salts and alkaline earth metal salts such as the calcium or magnesium salts.

In accordance with the present invention, the compounds may be formulated using one or more pharmaceutically acceptable excipients or carriers. Suitable formulations which may be used within the scope of the present invention have already been described in the literature and in patent applications related to these compounds. These formulations are incorporated herein by reference.

In one embodiment in accordance with the present invention, the formulation for the compound of formula A1 is a lipid suspension of the active substance comprising preferably a lipid carrier, a thickener and a glidant/solubilizing agent, most preferably in which the lipid carrier is selected from corn oil glycerides, diethylenglycolmonoethylether, ethanol, glycerol, glycofurol, macrogolglycerolcaprylocaprate, macrogolglycerollinoleate, medium chain partial glycerides, medium chain triglycerides, polyethylene glycol 300, polyethylene glycol 400, polyethylene glycol 600, polyoxyl castor oil, polyoxyl hydrogenated castor oil, propylene glycol monocaprylate, propylene glycol monolaurate, refined soybean oil, triacetin, triethyl citrate, or mixtures thereof, the thickener is selected from oleogel forming excipients, such as Colloidal Silica or Bentonit, or lipophilic or amphiphilic excipients of high viscosity, such as polyoxyl hydrogenated castor oil, hydrogenated vegetable oil macrogolglycerol-hydroxystearates, macrogolglycerol-ricinoleate or hard fats, and the glidant/solubilizing agent is selected from lecithin, optionally further comprising one or more macrogolglycerols, preferably selected from macrogolglycerol-hydroxystearate or macrogolglycerol-ricinoleate. The lipid suspension formulation may be prepared by conventional methods of producing formulations known from the literature, i.e. by mixing the ingredients at a pre-determined temperature in a pre-determined order in order to obtain a homogenized suspension.

The above formulation may be preferably incorporated in a pharmaceutical capsule, preferably a soft gelatin capsule, characterised in that the capsule shell comprises e.g. glycerol as plasticizing agent, or a hard gelatin or hydroxypropylmethylcellulose (HPMC) capsule, optionally with a sealing or banding. The capsule pharmaceutical dosage form may be prepared by conventional methods of producing capsules known from the literature. The soft gelatin capsule may be prepared by conventional methods of producing soft gelatin capsules known from the literature.

The above defined formulation or the above defined capsule may be used in a dosage range of from 0.1 mg to 20 mg of active substance/ kg body weight, preferably 0.5 mg to 4 mg active substance /kg body weight.

The above defined capsules may be packaged in a suitable glass container or flexible plastic container, or in an aluminium pouch or double poly bag.

The dosages and schedules may vary according to the particular disease state and the overall condition of the patient. Dosages and schedules may also vary if, in addition to a treatment with compound A of the present invention or a pharmaceutically acceptable salt thereof, one or more additional chemotherapeutic agents is/are used. Scheduling can be determined by the practitioner who is treating any particular patient.

Radiotherapy may be administered according to the known practices in clinical radiotherapy. The dosages of ionising radiation will be those known for use in clinical radiotherapy.

The size of the dose of each therapy which is required for the therapeutic or prophylactic treatment of a particular disease state will necessarily be varied depending on the host treated, the route of administration and the severity of the illness being treated. Accordingly the optimum dosage may be determined by the practitioner who is treating any particular patient. For example, it may be necessary or desirable to reduce the above-mentioned doses of the components of the combination treatments in order to reduce toxicity.

## Claims

1. Nintedanib for use in a method for the treatment of non-small cell lung cancer in a patient who has received prior treatment with an anti-tumour therapy other than with nintedanib, which method comprises treating said patient with a treatment regimen comprising the administration of nintedanib or a pharmaceutically acceptable salt thereof, wherein the patient to be treated is selected for treatment on the basis of having shown progression of the cancer within a period of 9 months or less after the initiation of said prior treatment.

2. Nintedanib for use in a method in accordance with claim 1, wherein said prior treatment with an anti-tumour therapy other than with nintedanib is a monotherapeutic treatment with a platinum compound, or a combination treatment of a platinum compound with one or more further therapeutic agents other than nintedanib, with or without adjuvant or neoadjuvant therapy.

3. Nintedanib for use in a method in accordance with claim 1, wherein the treatment regimen comprising the administration of nintedanib or a pharmaceutically acceptable salt thereof is a treatment regimen with nintedanib or nintedanib esylate, when used alone or in separate, simultaneous or sequential combination with further pharmaceutically active ingredients and/or further therapeutic treatments.

4. Nintedanib for use in a method in accordance with claim 1, wherein the treatment regimen comprising the administration of nintedanib or a pharmaceutically acceptable salt thereof is a combination treatment regimen with an anti-cancer drug from plants selected from docetaxel or paclitaxel, or a pharmaceutically acceptable salt thereof, or with an anti-folate selected from pemetrexed or pralatrexate, or a pharmaceutically acceptable salt thereof.

## Patentansprüche

1. Nintedanib zur Verwendung in einem Verfahren zur Behandlung von nichtkleinzelligem Lungenkrebs bei einem Patienten, der eine vorherige Behandlung mit einer anderen Anti-Tumortherapie als mit Nintedanib erhalten hat, wobei das Verfahren die Behandlung des Patienten mit einem Behandlungsplan umfasst, der die Verabreichung von Nintedanib oder eines pharmazeutisch akzeptablen bzw. annehmbaren Salzes hiervon umfasst, wobei der zu behandelnde Patient für die Behandlung auf der Basis ausgewählt wird, dass er innerhalb einer Zeitspanne von 9 Monaten oder weniger nach Beginn der vorherigen Behandlung eine Progression des Krebses gezeigt hat.

2. Nintedanib zur Verwendung in einem Verfahren nach Anspruch 1, wobei die vorherige Behandlung mit einer anderen Anti-Tumortherapie als mit Nintedanib eine monotherapeutische Behandlung mit einer Platinverbindung darstellt, oder eine Kombinationsbehandlung einer Platinverbindung mit einem oder mehreren weiteren therapeutischen Mitteln außer Nintedanib, mit oder ohne adjuvante oder neoadjuvante Therapie.

3. Nintedanib zur Verwendung in einem Verfahren nach Anspruch 1, wobei der Behandlungsplan, umfassend die Verabreichung von Nintedanib oder einem pharmazeutisch akzeptablen bzw. annehmbaren Salz hiervon, einen Behandlungsplan mit Nintedanib oder Nintedanib-Esylat darstellt, wenn es alleine oder in getrennter, gleichzeitiger oder aufeinanderfolgender Kombination mit weiteren pharmazeutisch wirksamen bzw. aktiven Bestandteilen und/oder weiteren therapeutischen Behandlungen verwendet wird.

4. Nintedanib zur Verwendung in einem Verfahren nach Anspruch 1, wobei der Behandlungsplan, umfassend die Verabreichung von Nintedanib oder einem pharmazeutisch akzeptablen bzw. annehmbaren Salz hiervon, einen Kombinationsbehandlungsplan mit einem Anti-Krebs-Arzneistoff aus Pflanzen darstellt, ausgewählt aus Docetaxel oder Paclitaxel, oder einem pharmazeutisch akzeptablen bzw. annehmbaren Salz hiervon, oder mit einem Antifolat, ausgewählt aus Pemetrexed oder Pralatrexat, oder einem pharmazeutisch akzeptablen bzw. annehmbaren Salz hiervon.

## Revendications

1. Nintédanib pour une utilisation dans une méthode pour le traitement du cancer du poumon non à petites cellules chez un patient qui a reçu un traitement antérieur avec une thérapie antitumorale autre qu'avec du nintédanib, laquelle méthode comprend le traitement dudit patient avec un régime thérapeutique comprenant l'administration de nintédanib ou de l'un de ses sels pharmaceutiquement acceptables, dans lequel le patient à traiter est sélectionné pour un traitement sur la base d'avoir présenté une progression du cancer dans une période de 9 mois ou moins après l'initiation dudit traitement antérieur.

2. Nintédanib pour une utilisation dans une méthode conformément à la revendication 1, **caractérisé en ce que** ledit traitement antérieur avec une thérapie antitumorale autre qu'avec du nintédanib est un traitement monothérapeutique avec un composé de platine, ou un traitement combiné d'un composé de platine avec un ou plusieurs autres agents thérapeutiques autres que le nintédanib, avec ou sans thérapie adjuvante ou néo-adjuvante.

3. Nintédanib pour une utilisation dans une méthode conformément à la revendication 1, **caractérisé en ce que** le régime thérapeutique comprenant l'administration de nintédanib ou de l'un de ses sels pharmaceutiquement acceptables est un régime thérapeutique avec du nintédanib ou de l'ésylate de nintédanib, lors d'une utilisation seul ou dans une combinaison séparée, simultanée ou séquentielle avec d'autres composants pharmaceutiquement actifs et/ou d'autres traitements thérapeutiques.

4. Nintédanib pour une utilisation dans une méthode conformément à la revendication 1, **caractérisé en ce que** le régime thérapeutique comprenant l'administration de nintédanib ou de l'un de ses sels pharmaceutiquement acceptables est un régime thérapeutique combiné avec un médicament anticancéreux issu de plantes sélectionné parmi le docétaxel ou le paclitaxel, ou l'un de ses sels pharmaceutiquement acceptables, ou avec un antifolate sélectionné parmi le pémétrexed ou le pralatrexate, ou l'un de ses sels pharmaceutiquement acceptables.
